# EUROPEAN PATENT SPECIFICATION

(11) **EP 0 969 293 B1**
(45) Date of publication and mention of the grant of the patent: **26.02.2003**
(21) Application number: 98305168.1
(22) Date of filing: 30.06.1998
(51) Int. Cl.: G01T 1/164, G01T 1/29

(54) **Nuclear camera system using radio-isotopes**
Nuklearkamerasystem unter Verwendung von Radio-isotopen
Appareil photographique nucléaire utilisant des radio-isotopes

(43) Date of publication of application: 05.01.2000
(73) Proprietor: Marconi Medical Systems, Inc., Highland Heights, Ohio 44143 (US)
(72) Inventor: Gagnon, Daniel, Mayfield Heights, Ohio 44124 (US); Difilippo, Frank P., University Heights Ohio 44118 (US)
(74) Representative: van der Veer, Johannis Leendert

(56) References cited:
- WO-A-92/18881
- WO-A-97/20232
- US-A- 5 600 145
- US-A- 5 610 402

## Description

The present invention relates to a nuclear or gamma camera for diagnostic imaging.

It is to be appreciated, however, that the present invention will also find application in other non-invasive investigation techniques and imaging systems such as single photon planar imaging, whole body nuclear scans, positron emission tomography (PET) and other diagnostic modes.

Positron emission tomography (PET) scanners are known as coincidence imaging devices. In planar coincidence imaging, two radiation detectors oppose each other with a subject disposed between the detectors. Typically, one or more radiopharmaceuticals or radioisotopes capable of generating positron emission radiation are injected into the subject. The radioisotope preferably travels to an organ of interest whose image is to be produced. The detectors scan the subject along a longitudinal axis without rotation producing a data set with incomplete angular sampling, otherwise known as limited angle tomography. Radiation events are detected on each detector and a coincidence circuitry compares and temporally matches the events on each detector. Events on one detector which have a coincident event on the other detector are treated as valid data and may be used in image reconstruction. Document US-A-5 600 145 describes such an arrangement.

Typically, the detector includes a scintillation crystal that is viewed by an array of photomultiplier tubes. The relative outputs of the photomultiplier tubes are processed and corrected, as is conventional in the art, to generate an output signal indicative of (1) a position coordinate on the detector head at which each radiation event is received, and (2) an energy of each event. The energy is used to differentiate between various types of radiation such as multiple emission radiation sources and to eliminate noise, or stray and secondary emission radiation. A two-dimensional image representation is defined by the number of coincidence radiation events or counts received at each coordinate. However during a scan, only a fraction of the events detected are coincidence events. As such, scan times are increased in an effort to obtain a sufficient data sampling for image reconstruction which poses additional inconveniences to the subject and an increase in scanning costs from reduced patient throughput.

In accordance with the present invention, a new and improved diagnostic imaging system and method for diagnostic imaging is provided. The diagnostic imaging system includes a gantry which defines an examination region that receives a subject where the subject includes a positron emitter and a single photon emitter. First and second radiation detectors are oppositely disposed on the gantry and have the examination region therebetween. The first and second radiation detectors detect radiation from the examination region. A coincidence circuit is connected to the first and second radiation detectors and determines coincidence radiation events emitted from the positron emitter. Coincidence data is generated based on the coincidence radiation events. A third radiation detector which includes a collimator, detects collimated radiation travelling along a selected projection path. The third radiation detector is supported on the gantry at an angle to the first and second radiation detectors. A projection data processor is connected to the third radiation detector and generates collimated projection data based on collimated radiation detected from the single photon emitter. A combiner selectively combines the coincidence data and the collimated projection data into an image volume and a reconstruction processor reconstructs an image representation from the image volume.

In a more limited aspect of the present invention, the diagnostic imaging system further includes a transmission radiation source which generates transmission radiation toward the examination region. The third radiation detector detects both the transmission radiation from the transmission radiation source and emission radiation from the subject. A sorter sorts the emission and transmission radiation detected. The projection data processor generates transmission projection data based on the transmission radiation detected and selectively combines the transmission projection data with the collimated projection data.

In accordance with another aspect of the present invention, a diagnostic imaging system is provided including a gantry which supports a plurality of radiation detectors which detect coincidence radiation emitted from a subject disposed in an examination region. A processor generates coincidence data from the detected coincidence radiation and a reconstruction processor reconstructs the coincidence data into an image representation of a selected portion of the subject. The diagnostic imaging system further includes a collimated radiation detector which detects collimated radiation from the examination region. A collimation data processor generates collimated radiation data based on the collimated radiation detected and the collimated radiation data is selectively combined with the coincidence data before reconstruction by the reconstruction processor.

Diagnostic imaging systems constructed in accordance with the invention will now be described, by way of example, with reference to the accompanying drawings in which:
Figure 1 is a diagrammatic illustration of a diagnostic imaging system in accordance with the present invention;
Figure 2 is an illustration of one embodiment of a coincidence radiation detector system and a collimated radiation detector system in accordance with the present invention;
Figure 3 is an example of an alternative embodiment of the present invention;
Figure 4 is an example of a diagnostic system to simultaneously collect data from coincidence PET and SPECT images in accordance with the present invention;
Figure 5 is an example of another alternative embodiment of the present invention;
Figure 6 is an illustration of another alternative embodiment which includes one or more transmission radiation sources;
Figure 7 illustrates a line source configuration in accordance with the present invention.

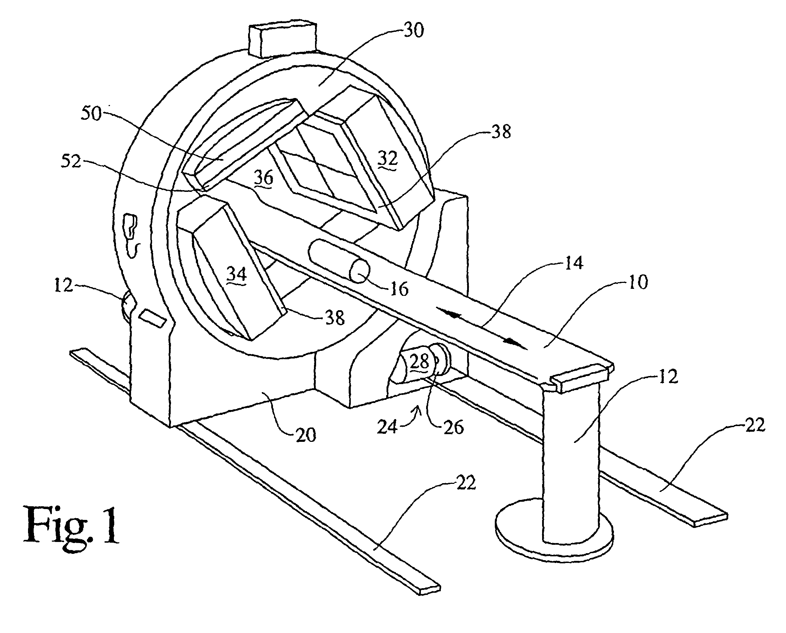

With reference to Figure 1, a diagnostic imaging system includes a subject support or table **10** which is mounted to stationary, vertical supports **12** at opposite ends. The subject table is selectively positionable up and down to centre a subject **16** in the centre of a circle along a longitudinal axis **14.**

An outer gantry structure **20** is movably mounted on tracks **22** which extend parallel to the longitudinal axis. This enables the outer gantry structure to be moved parallel to the longitudinal axis **14.** An outer gantry structure moving assembly **24** is provided for selectively moving the outer gantry structure **20** along the tracks **22** in a path parallel to the longitudinal axis. In the illustrated embodiment, the longitudinal moving assembly includes drive wheels **26** for supporting the outer gantry structure on the tracks. A motive power source, such as a motor **28,** selectively drives one of the wheels which frictionally engages the track and drives the outer gantry structure and supported inner gantry structure and detector heads therealong. Alternately, the outer gantry can be stationary and the subject support configured to move the subject along the longitudinal axis.

An inner gantry structure **30** is rotatably mounted on the outer gantry structure **20.** A first camera or radiation detector head **32** is mounted to the inner gantry structure. A second radiation detector head **34** is mounted to the inner gantry structure opposite to the first camera head. The first and second detectors **32, 34** are configured to detect positron annihilation radiation generated by a positron emission source injected into the subject. The inner gantry structure defines a central, subject receiving examination region **36** for receiving the subject table and, particularly along the longitudinal axis. The examination region **36** is enlarged to receive the detector heads in any of a variety of displacements from a central axis and angular orientations.

The detectors each include a scintillation crystal disposed behind a radiation receiving face **38** that is viewed by an array of photomultiplier tubes. The scintillation crystal emits a flash of light in response to incident radiation. The array of photomultiplier tubes convert the light into electrical signals. A resolver circuit resolves the x,y-coordinates of each light flash and the energy of the incident radiation. The relative outputs of the photomultiplier tubes are processed and corrected, as is conventional in the art, to generate an output signal indicative of a position coordinate on the detector head at which each radiation event is received, and an energy of each event.

A coincidence data processor or coincidence imaging subsystem **40** collects **42** the position coordinates and energy values obtained based on the radiation events detected by each detector **32** and **34.** A coincidence circuitry **44** compares and matches radiation events from positron emissions **58** generated by a positron emitter within the subject which were coincidentally detected by the detectors **32** and **34.** Based on the coincidence events, the coincidence data processor generates coincidence data which is stored in a coincidence memory **46.**

The diagnostic imaging system includes a third radiation detector head **50** supported by the gantry which is disposed at an angle to the coincidence radiation detectors **32, 34.**

The third detector is configured to acquire projection radiation data and includes a collimator **52** mounted on a front face to restrict received radiation to radiation travelling generally perpendicular to the face. Of course, various types of collimators can be used to obtain a desired projection path such as a parallel beam, a cone beam or fan beam geometry. The third detector **50** includes a scintillation crystal that is viewed by an array of photomultiplier tubes. The relative outputs of the photomultiplier tubes are processed and corrected to generate an output signal indicative of a position coordinate on the detector at which each radiation event is received, and an energy of each event.

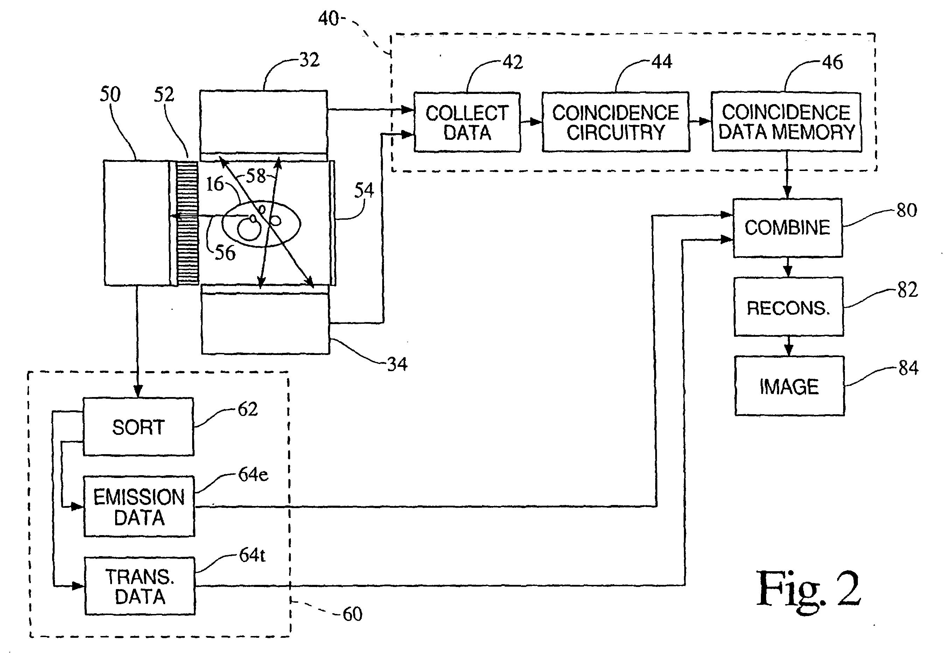

With reference to Figure 2, the diagnostic imaging system includes a transmission radiation source **54** disposed across from the third radiation detector with the examination region **36** disposed therebetween. The transmission radiation source transmits radiation having a different energy level than the injected radioisotopes throughout the examination region which is detected by the third radiation detector. However, the'transmission radiation may have the same energy level as the emission radiation in which case the radiation is differentiated by the positions on the detector. In the preferred embodiment, the collimator is a high energy collimator capable of handling 511keV gamma radiation and limits the radiation received by the third radiation detector to be projections normal to the coincidence geometry. With the transmission radiation source, the third radiation detector receives both transmission and emission radiation.

With further reference to Figure 2, an exemplary single photon data processor **60** is provided for the collimated third detector **50.** Both 511keV positron annihilation radiation emitted from the subject and transmission radiation from the transmission radiation source **54** is received by the third detector **50.** A sorter **62** sorts the emission projection data and the transmission projection data based on the energy of the detected radiation. The data may also be sorted based solely on the location of the detected event, or based on a combination of energy and location. The sorted data are stored in a projection view memory **64,** more specifically in corresponding emission data memory **64e** and transmission data memory **64t.**

The collimated projection data **64e, 64t** obtained is particularly useful in rapid whole-body limited angle tomography. The collimated data **64e, 64t** is optimally obtained simultaneously with the coincidence events. Thus, the diagnostic imaging system is capable of operating a variety of detectors in different modes. A combiner **80** combines the information from the collimated data **64e, 64t** with the coincidence data **46** in several different ways in order to supplement the coincidence data **46.** In one embodiment, the collimated data **64e, 64t** is used to define a boundary outline of a region of interest within the subject so as to limit the range of reconstruction required for a limited angle tomography. Alternately, the collimated data **64e, 64t** is used to establish the number of counts per plane which is used to further constraint the reconstruction of the coincidence data **46** so that information unrelated to the region of interest is not reconstructed. In another alternative, the collimated information **64e, 64t** is reconstructed together with the coincidence data **46.** A reconstruction processor **82** reconstructs the combined coincidence and collimated data into an image representation **84,** for example, by a blind deconvolution-type technique which reconstructs different types of data. An example of a blind deconvolution technique is discussed in "Blind Image Deconvolution," by Kundur and Hatzinkos, IEEE Signal Processing Magazine, Vol.13, No. 3, page 43, 1996.

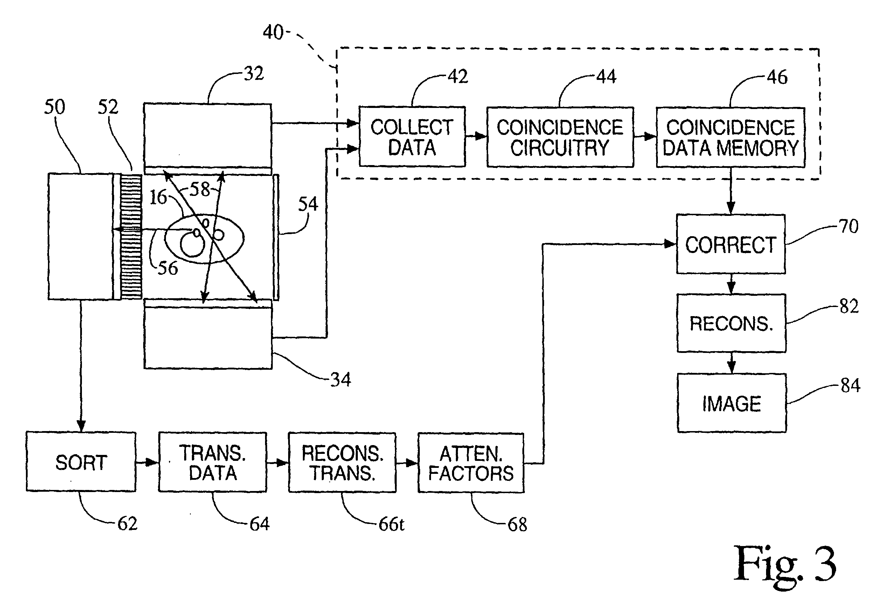

With reference to Figure 3, the diagnostic imaging system may also be used to produce transmission attenuation corrected PET images. As the gantry and hence the detectors **32, 34, 50** are rotated about the patient, data received by the detectors **32, 34** is collected **42** and processed by the coincidence circuitry **40** as described above. Valid coincidence events are stored in a coincidence projection view memory **46.** Simultaneously, transmission radiation from the transmission radiation source **54** is received by the third detector **50.** A sorter **62** selects the transmission data based on energy of detected radiation, discarding detected events which do not correspond in energy to that of the transmission radiation source **54.** Alternately, the data may be sorted based on the position of the detected event, either alone or in combination with its energy. The data are stored in a transmission projection view memory **64.**

The coincidence data normally contains inaccuracies caused by varying absorption characteristics of the patient's anatomy. A reconstruction processor **66t** reconstructs the transmission data into a transmission image representation of volume attenuation factors stored in a memory **68.** Each voxel value is indicative of attenuation of tissue in a corresponding location within the patient. A coincidence data correction means **70** corrects the coincidence data in accordance with the attenuation factors determined from the transmission data. More specifically, for each ray along which coincidence data is received, the correction means calculates a corresponding ray through the transmission attenuation factors. Each ray of the coincidence data is then weighted or corrected **70** in accordance with the attenuation factors and reconstructed by a coincidence radiation reconstruction processor **82** to generate a three-dimensional coincidence image representation **84** of the patient.

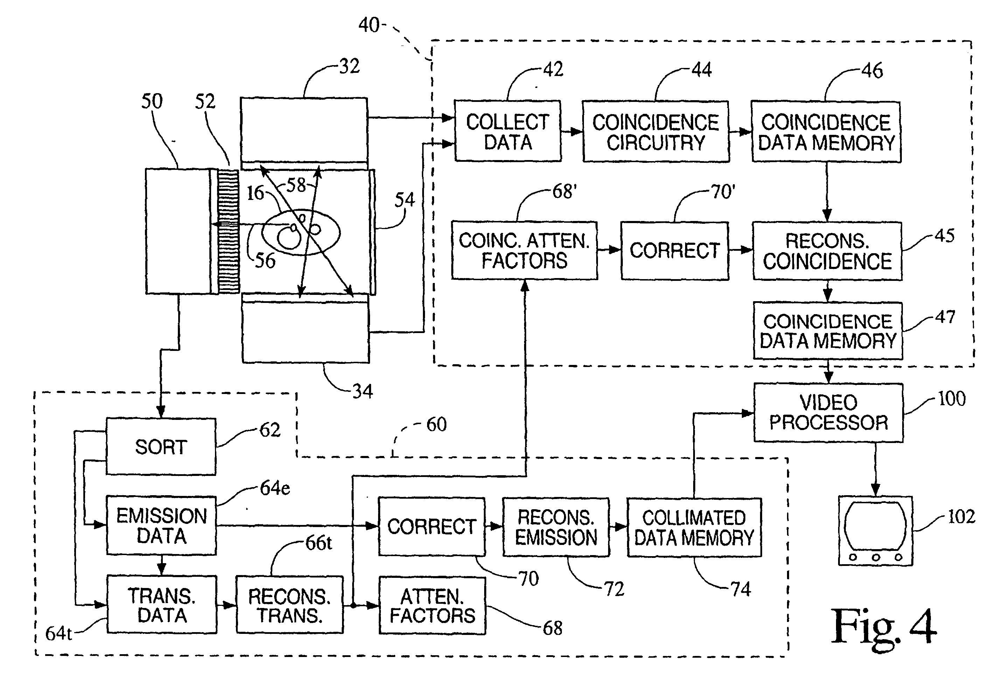

With reference to Figure 4, the diagnostic imaging system may also be used to simultaneously collect data for coincidence PET and SPECT images in dual isotope imaging. For example, a radiopharmaceutical which produces positron radiation (e.g., 18 F-FDG) and a radiopharmaceutical which produces single photon emission radiation (e.g., ^{99m.}Tc-MIBI) may both be introduced into the subject.

As the gantry and hence the detectors **32, 34, 50** are rotated about the patient, data received by the detectors **32, 34** is collected **42** and processed by the coincidence circuitry **44.** Valid coincidence events are stored in a coincidence projection view memory **46.** Simultaneously, single photon emission data from the subject and transmission radiation from the transmission radiation source **54** are received by the third detector **50.** A sorter **62** sorts the emission projection data and the transmission projection data on the basis of relative energies. Alternately, the data may be sorted based on the position of the detected event either alone or in combination with the energy of the event. The data are stored in a projection view memory **64,** more specifically in emission data memory **64e** and transmission data memory **64t.**

The emission data normally contains inaccuracies caused by varying absorption characteristics of the patient's anatomy. A reconstruction processor **66t** reconstructs the transmission data into a transmission image representation of volume attenuation factors stored in a memory **68.** Each voxel value is indicative of attenuation of tissue in a corresponding location within the patient. An emission data correction means **70** corrects the emission data in accordance with the emission data attenuation factors **68** determined from the transmission data. More specifically, for each ray along which emission data is received, the emission correction means calculates a corresponding ray through the transmission attenuation factors. Each ray of the emission data is then weighted or corrected **70** in accordance with the attenuation factors and reconstructed by an emission radiation reconstruction processor **72** to generate a three-dimensional emission image representation that is stored in a collimated data memory **74.** Alternately, the corrected emission data is directly stored in the collimated data memory **74** without reconstruction.

The coincidence data may also contain inaccuracies caused by varying absorption characteristics of the patient's anatomy. Accordingly, a coincidence data correction means **70'** corrects the coincidence data in accordance with coincidence data attenuation factors **68'.** Figure 4 depicts the coincidence attenuation factors as being different from those for the emission data **68,** reflecting the different energy of the respective radiation. Alternately, the same correction factors may be used. A coincidence reconstruction processor **45** reconstructs the coincidence data to generate a three-dimensional coincidence image representation that is stored in a coincidence data memory **47.** Selected portions of the coincidence image **47** and the emission image **74** may then be processed by video processor **100** and displayed on video monitor **102** or in other suitable human readable form. Hence, a single diagnostic imaging system may be used to simultaneously generate attenuation corrected coincidence PET and attenuation corrected SPECT images.

In the event that attenuation correction of the emission data, the coincidence data, or both is not required, the associated attenuation correction processing may be deleted.

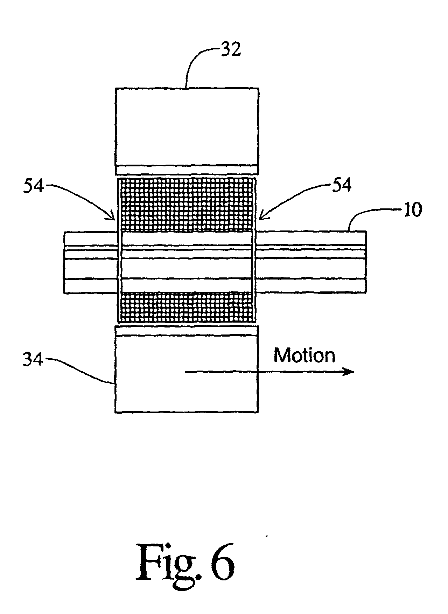

In yet another alternative embodiment and with reference to Figure 6, one or more fixed collimated transmission radiation sources **54** are disposed on the gantry. Radiation detection areas of the collimated radiation detector **50** only in front of the radiation sources **54** are used to acquire the transmission data while the remaining areas of the collimated radiation detector **50** are used to acquire only emission radiation while the first and second radiation detectors **32, 34** continue to operate in detecting coincidence events. In this way, transmission data may be collected for various portions of the anatomy as the gantry and patient are moved relative to each other. Thus, a drive mechanism for scanning or sweeping the transmission source or sources **54** across the face of the third detector **50** is not required.

Alternatively, a transmission radiation line source **54** may be moveably mounted to one of the detectors **32, 34** such that the line source **54** may be scanned or swept across the face of the detector **50.** The collimated radiation detector **50** acquires both transmission and emission data in coordination with the movement of the line source **54.**

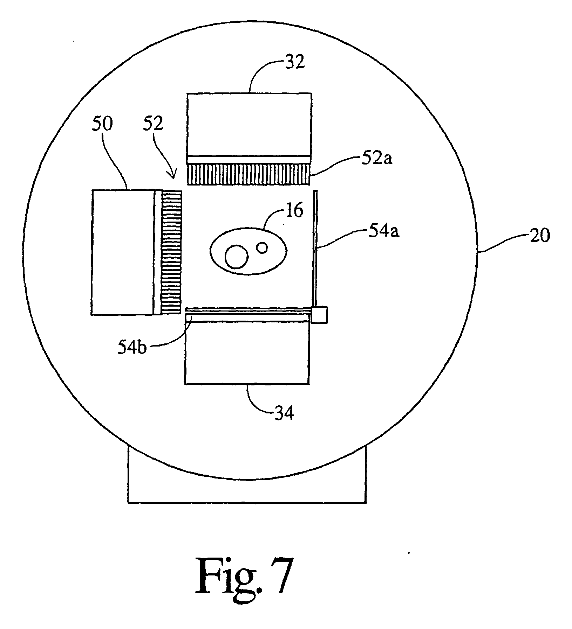

A line source configuration which is particularly advantageous in connection with cardiac SPECT imaging is shown in Figure 7. In this configuration, the detector **50** is fitted with a collimator **52** and the detector **32** is fitted with a collimator **52a.** Data from both detectors is collected as traditional single photon data. Two transmission radiation sources **54a, 54b** are moveably mounted to the detector **34** such that the transmission sources **54a, 54b** may be scanned or swept across the face of the detectors **50** and **32,** respectively. The transmission source **54a** is generally parallel to the face of the detector **50,** while the transmission source **54b** is perpendicular to the transmission source **54a** and generally parallel to the face of the detector **32.** Such an arrangement facilitates efficient generation of attenuation corrected cardiac SPECT data. Preferably, the line source **54a** and the collimator **52a** are each mounted so as to be readily installed in and removed from their respective mounting positions. With the line source **54a** and the collimator **52a** removed, the imaging device can be readily converted to collect coincidence and collimated data as described above.

Those skilled in the art will recognize that exemplary reconstruction technique for emission and transmission data have been described. Of course, the reconstruction technique varies according to the type and energy of the radiation collected and type of collimator used (i.e., fan, cone, parallel beam). Even using the same type and energy and collimator type, there are various possible reconstruction techniques, producing different image qualities at different execution speeds, as is well-known in the art.

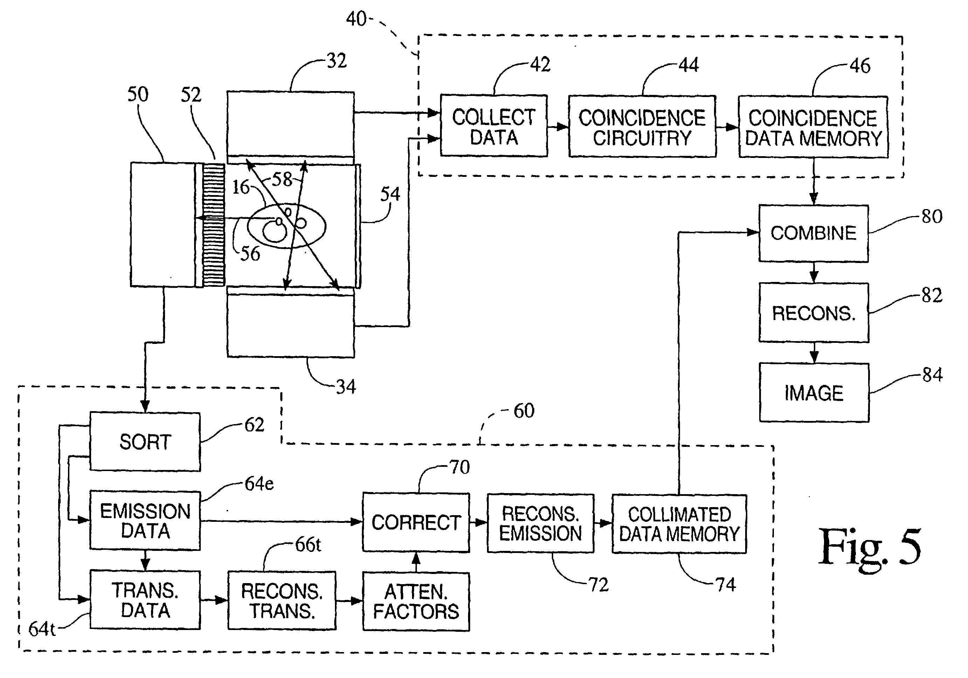

With reference to Figure 5, an exemplary single photon data processor or single photon imaging subsystem **60** is provided for the collimated third detector **50** which detects single photon radiation **56.** Furthermore, an exemplary reconstruction technique for emission and transmission data is provided. Of course, the reconstruction technique changes according to the types of radiation collected and the types of collimators used (i.e., fan, cone, parallel beam). Both emission radiation from the subject and transmission radiation from the transmission radiation source **54** is received by the third detector **50** and emission projection data is generated. The emission data normally contains inaccuracies caused by varying absorption characteristics of the subject's anatomy. A sorter **62** sorts the emission projection data and transmission projection data on the basis of the relative energies. The data are stored in a projection view memory **64,** more specifically in corresponding emission data memory **64e** and transmission data memory **64t.** A reconstruction processor **66t** reconstructs the transmission data into a transmission image representation or volume of attenuation factors stored in a memory **68.** Each voxel value stored in the memory **68** is indicative of attenuation of tissue in a corresponding location within the patient. An emission data correction means **70** corrects the emission data in accordance with the attenuation factors determined from the transmission data. More specifically, for each ray along which emission data is received, the emission correction means calculates a corresponding ray through the transmission attenuation factors stored in the memory **68.** Each ray of the emission data is then weighted or corrected **70** in accordance with the attenuation factors and reconstructed by an emission radiation reconstruction processor **62** to generate a three-dimensional emission image representation that is stored in a collimated data memory **74.** Alternately, the corrected emission data is directly stored in the collimated data memory without reconstruction.

The collimated projection data **74** obtained is particularly useful in rapid whole-body limited angle tomography. The collimated data **74** is optimally obtained simultaneously with the coincidence events. Thus, the diagnostic imaging system is capable of operating a variety of detectors in different modes. A combiner **80** combines the information from the collimated data **74** with the coincidence data **46** in several different ways in order to supplement the coincidence data **46.** In one embodiment, the collimated data **74** is used to define a boundary outline of a region of interest within the subject so as to limit the range of reconstruction required for a limited angle tomography. Alternately, the collimated data **74** is used to establish the number of counts per plane which is used to further constraint the reconstruction of the coincidence data **46** so that information unrelated to the region of interest is not reconstructed. In another alternative, the collimated information **74** is reconstructed together with the coincidence data **46.** A reconstruction processor **82** reconstructs the combined coincidence and collimated data into an image representation **84,** for example, by a blind deconvolution-type technique which reconstructs different types of data. An example of a blind deconvolution technique is discussed in "Blind Image Deconvolution," by Kundur and Hatzinkos, IEEE Signal Processing Magazine, Vol.13, No. 3, page 43, 1996.

Those skilled in the art will recognize that the present invention facilitates the combination of coincidence and single photon projection data. In particular, the detector and source configuration described above is particularly useful for producing whole body planar coincidence images with additional depth information, PET images with transmission attenuation correction, and combined PET/SPECT dual isotope imaging, either with or without transmission attenuation correction.

With the use of a stationary source and by moving the detector during a scan, attenuation information is obtained. Alternately, two or more stationary sources are disposed at each end of the third detector and the third detector is moved during a scan. This decreases "ramp-up" distance for the attenuation information when part of the patient is in the field of view when scan starts and/or ends.

A video processor withdraws selected portions of the data from the reconstructed image to generate corresponding human-readable displays on a video monitor. Typical displays include reprojections, selected slices or planes, surface renderings, whole-body tomographic images and the like.

Among advantages of the described embodiments are that a positron imaging system which generates coincidence events is combined with a single photon imaging system; image reconstruction is improved by combining coincidence data with collimated data; sufficient image quality may be obtained in a shorter scan time, thereby improving patient throughput and minimizing patient inconvenience; the production of whole body planar images with additional depth information, PET images with transmission attenuation, and combined PET/SPECT, dual isotope imaging is facilitated.

The invention has been described with reference to the preferred embodiments. Obviously, modifications and alterations will occur to others upon reading and understanding the preceding detailed description. It is intended that the invention be construed as including all such modifications and alterations insofar as they come within the scope of the appended claims or the equivalents thereof.

## Claims

1. A nuclear camera system comprising: a gantry defining an examination region for receiving a subject, the subject including one of (i) a positron emitter and (ii) a positron emitter and a single photon emitter; first and second radiation detectors oppositely disposed on the gantry and having the examination region therebetween, the first and second radiation detectors detecting radiation from the examination region; a coincidence circuit connected to the first and second radiation detectors for determining coincidence radiation events emitted from the positron emitter and generating coincidence data based on the coincidence radiation events; a third radiation detector including a collimator for detecting collimated radiation travelling along a selected projection path, the third radiation detector being supported on the gantry at an angle to the first and second radiation detectors; a projection data processor connected to the third radiation detector for generating collimated projection data based on collimated radiation detected from the single photon emitter; a combiner which selectively combines the coincidence data and the collimated projection data into an image volume; and a reconstruction processor for reconstructing an image representation from the image volume.

2. A nuclear camera system as claimed in claim 1, further including: a transmission radiation source for generating transmission radiation toward the examination region, the third radiation detector detecting both the transmission radiation from the transmission radiation source and emission radiation from the subject; a sorter connected to the third radiation detector for sorting the emission radiation and the transmission radiation detected; and the projection data processor generating transmission projection data based on the transmission radiation detected and selectively combining the transmission projection data with the collimated projection data.

3. A nuclear camera system as claimed in claim 1 or claim 2, further including: a motor assembly for selectively moving the gantry along a non-rotating path along a longitudinal axis.

4. A nuclear camera system as claimed in claim 3, wherein the motor assembly selectively rotates the gantry around the examination region.

5. A method of diagnostic imaging with a nuclear camera system including an examination region having a subject disposed therein, the method comprising: injecting the subject with first and second isotopes, the first isotope generating positron emission radiation and the second isotope generating single photon emission radiation; detecting coincidence radiation events from the positron emission radiation; generating coincidence data based on the coincidence radiation events detected; concurrently with the step of detecting coincidence radiation events, detecting single photon emissions from the single photon emission radiation; generating single photon emission data based on the single photon emissions detected; combining the coincidence data and the single photon emission data into an image volume; and reconstructing an image representation of the subject from the image volume.

6. A method of diagnostic imaging as claimed in claim 5, further including: transmitting transmission radiation through the examination region; detecting the transmission radiation and generating transmission data based on the transmission radiation detected; and combining the transmission data, the single photon emission data and the coincidence data into the image volume.

7. A method of diagnostic imaging as claimed in claim 5, wherein the reconstructing includes blind deconvolution.

## Patentansprüche

1. Nuklearkamerasystem das Folgendes umfasst: eine Gantry, die einen Untersuchungsbereich definiert, der ein Objekt aufnimmt, wobei das Objekt (i) einen Positronenstrahler und (ii) einen Positronenstrahler und einen Einzelphotonenstrahler umfasst: einen ersten und einen zweiten Strahlungsdetektor, die einander gegenüberliegend auf der Gantry angeordnet sind, so dass die Untersuchungsregion zwischen den beiden Detektoren liegt, wobei der erste und der zweite Strahlungsdetektor die Strahlung von der Untersuchungsregion erkennen; eine Koinzidenzschaltung, die mit dem ersten und dem zweiten Strahlungsdetektor verbunden ist, um die von dem Positronenstrahler emittierten koinzidenten Strahlungsereignisse zu ermitteln und ausgehend von den koinzidenten Strahlungsereignissen Koinzidenzdaten zu generieren; einen dritten Strahlungsdetektor, der einen Kollimator enthält, um die kollimierte Strahlung, die sich entlang eines gewählten Projektionspfades fortpflanzt, zu erkennen, wobei der dritte Strahlungsdetektor auf der Gantry in einem Winkel zu dem ersten und dem zweiten Strahlungsdetektor angebracht ist; einen Projektionsdatenprozessor, der mit dem dritten Strahlungsdetektor verbunden ist, um basierend auf der erkannten kollimierten Strahlung vom Einzelphotonenstrahler kollimierte Projektionsdaten zu erzeugen; einen Kombinierer, der selektiv die Koinzidenzdaten und die kollimierten Projektionsdaten zu einem Bildvolumen kombiniert; und einen Rekonstruktionsprozessor, der aus dem Bildvolumen eine Bilddarstellung rekonstruiert.

2. Nuklearkamerasystem nach Anspruch 1, das weiterhin Folgendes umfasst: eine Transmissionsstrahlungsquelle zum Erzeugen einer auf die Untersuchungsregion gerichteten Transmissionsstrahlung, wobei der dritte Strahlungsdetektor sowohl die Transmissionsstrahlung von der Transmissionsstrahlungsquelle als auch die Emissionsstrahlung vom Objekt erkennt; einen Sortierer, der mit dem dritten Strahlungsdetektor verbunden ist und die erkannte Emissions- und Transmissionsstrahlung sortiert; und den Projektionsdatenprozessor, der Transmissionsprojektionsdaten ausgehend von der erkannten Transmissionsstrahlung erzeugt und die Transmissionsprojektionsdaten selektiv mit den kollimierten Projektionsdaten kombiniert.

3. Nuklearkamerassystem nach Anspruch 1 oder 2, das weiterhin Folgendes umfasst: eine Motorbaugruppe zum selektiven Bewegen der Gantry auf einem nichtrotierenden Weg entlang einer Längsachse.

4. Nuklearkamerasystem nach Anspruch 3, wobei die Motorbaugruppe die Gantry selektiv um die Untersuchungsregion dreht.

5. Verfahren der Diagnose-Bildgebung mit einem Nuklearkamerasystem mit einer Untersuchungsregion, in der ein Objekt angeordnet ist, wobei das Verfahren Folgendes umfasst: Injizieren eines ersten und eines zweiten Isotops in das Objekt, wobei das erste Isotop eine Positronenemissionsstrahlung erzeugt und das zweite Isotop eine Einzelphotonenemissionsstrahlung erzeugt; Erkennen von koinzidenten Strahlungsereignissen von der Positronenemissionsstrahlung; Erzeugen von Koinzidenzdaten basierend auf den erkannten koinzidenten Strahlungsereignissen; gleichzeitig mit dem Schritt des Erkennens von koinzidenten Strahlungsereignissen Erkennen von Einzelphotonenemissionen von der Einzelphotonenemissionsstrahlung; Erzeugen von Einzelphotonenemissionsdaten basierend auf den erkannten Einzelphotonenemissionen; Kombinieren der Koinzidenzdaten und der Einzelphotonenemissionsdaten zu einem Bildvolumen; und Rekonstruieren eines Bilddarstellung des Objekts aus dem Bildvolumen.

6. Verfahren der Diagnose-Bildgebung nach Anspruch 5, das weiterhin Folgendes umfasst: Aussenden von Transmissionsstrahlung durch die Untersuchungsregion; Erkennen der Transmissionsstrahlung und Erzeugen von Transmissionsdaten basierend auf der erkannten Transmissionsstrahlung; und Kombinieren der Transmissionsdaten, der Einzelphotonenemissionsdaten und der Koinzidenzdaten zu dem Bildvolumen.

7. Verfahren der Diagnose-Bildgebung nach Anspruch 5, wobei die Rekonstruktion eine blinde Dekonvolution umfasst.

## Revendications

1. Système à caméra nucléaire comprenant un portique définissant une zone d'examen pour recevoir un sujet, le sujet comprenant un (i) émetteur de positrons ou (ii) un émetteur de positrons et un émetteur monophotonique, des premier et deuxième détecteurs de rayonnement disposés l'un en face de l'autre sur le portique et entre lesquels se trouve la zone d'examen, les premier et deuxième détecteurs de rayonnement détectant un rayonnement à partir de la zone d'examen, un circuit à coïncidence connecté aux premier et deuxième détecteurs de rayonnement pour déterminer des événements de rayonnement à coïncidence émis de l'émetteur de positrons et générant des données de coïncidence sur la base des événements de rayonnement à coïncidence, un troisième détecteur de rayonnement comprenant un collimateur pour détecter un rayonnement collimaté circulant suivant un chemin de projection déterminé, le troisième détecteur de rayonnement étant supporté sur le portique en formant un angle par rapport aux premier et deuxième détecteurs de rayonnement, un dispositif de traitement de données de projection connecté au troisième détecteur de rayonnement pour générer des données de projection collimatées sur la base du rayonnement collimaté détecté à partir de l'émetteur monophotonique, un combineur qui combine de manière sélective les données de coïncidence et les données de projection collimatées en un volume image, et un dispositif de traitement de reconstruction pour reconstruire une représentation d'image à partir du volume image.

2. Système à caméra nucléaire suivant la revendication 1, comprenant en outre une source de rayonnement de transmission pour générer un rayonnement de transmission en direction de la zone d'examen, le troisième détecteur de rayonnement détectant à la fois le rayonnement de transmission à partir de la source de rayonnement de transmission et le rayonnement d'émission à partir du sujet ; un dispositif de tri connecté au troisième détecteur de rayonnement pour trier le rayonnement d'émission et le rayonnement de transmission détectés, et le dispositif de traitement de données de projection générant des données de projection de transmission sur la base du rayonnement de transmission détecté et combinant de manière sélective les données de projection de transmission et les données de projection collimatées.

3. Système à caméra nucléaire suivant la revendication 1 ou 2, comprenant en outre un ensemble moteur pour déplacer de manière sélective le portique suivant un chemin non rotatif suivant un axe longitudinal.

4. Système à caméra nucléaire suivant la revendication 3, dans lequel l'ensemble moteur tourne de manière sélective le portique autour de la zone d'examen.

5. Procédé d'imagerie diagnostique avec un système de caméra nucléaire comprenant une zone d'examen dans laquelle est disposé un sujet, le procédé comprenant l'injection dans le sujet de premier et de deuxième isotopes, le premier isotope générant un rayonnement d'émission de positrons et le deuxième isotope générant un rayonnement d'émission monophotonique ; la détection d'événements de rayonnement à coïncidence à partir du rayonnement d'émission de positrons ; la génération de données de coïncidence sur la base des événements de rayonnement à coïncidence détectés ; de manière concourante à l'étape de détection des événements de rayonnement à coïncidence, la détection des émissions monophotoniques du rayonnement d'émission monophotonique ; la génération de données d'émission monophotonique sur la base des émissions monophotoniques détectées ; la combinaison des données de coïncidence et des données d'émission monophotonique en un volume image, et la reconstruction d'une représentation d'image du sujet à partir du volume image.

6. Procédé d'imagerie diagnostique suivant la revendication 5, comprenant en outre la transmission d'un rayonnement de transmission à travers la zone d'examen ; la détection du rayonnement de transmission et la génération de données de transmission sur la base du rayonnement de transmission détecté, et la combinaison des données de transmission, des données d'émission monophotonique et des données de coïncidence dans le volume image.

7. Procédé d'imagerie diagnostique suivant la revendication 5, dans lequel la reconstruction comprend une déconvolution aveugle.
